(19) Europäisches Patentamt
European Patent Office
Office européen des brevets

(11) Veröffentlichungsnummer : **0 141 053**
**B1**

(12) # EUROPÄISCHE PATENTSCHRIFT

(45) Veröffentlichungstag der Patentschrift : **19.08.87**

(51) Int. Cl.⁴ : **C 07 D263/58**

(21) Anmeldenummer : **84108660.6**

(22) Anmeldetag : **21.07.84**

(54) **Verfahren zur Herstellung von 2-Chlorbenzoxazolen.**

(30) Priorität : **23.09.83 DE 3334417**

(43) Veröffentlichungstag der Anmeldung : **15.05.85 Patentblatt 85/20**

(45) Bekanntmachung des Hinweises auf die Patenterteilung : **19.08.87 Patentblatt 87/34**

(84) Benannte Vertragsstaaten : **BE CH DE FR GB IT LI NL**

(56) Entgegenhaltungen :
**EP-A- 0 043 573**
**DE-A- 3 207 153**
**R.C. ELDERFIELD "Heterocyclic Compounds", Band 5, 1957, JOHN WILLY & SONS INC. New York, Seiten 447,448**
**BEILSTEINS HANDBUCH DER ORGANISCHEN CHEMIE, 4. Auflage, Band 27, 1937, VERLAG SPRINGER, Berlin, Seite 43**
**BEILSTEINS HANDBUCH DER ORGANISCHEN CHEMIE, 4. Auflage, 3. und 4. Ergänzungswerk, Band 27, 2. Teil, 1983, VERLAG SPRINGER, Berlin, Seite 1069**

(73) Patentinhaber : **CASSELLA Aktiengesellschaft**
**Hanauer Landstrasse 526**
**D-6000 Frankfurt am Main 61 (DE)**

(72) Erfinder : **Becherer, Johannes, Dr.**
**Weidenseestrasse 8**
**D-6457 Maintal-Bischofsheim (DE)**
Erfinder : **Handte, Reinhardt, Dr.**
**Theilweg 23**
**D-8901 Gablingen (DE)**
Erfinder : **Nestler, Hans-Jürgen, Dr.**
**Steinweg 15**
**D-6240 Königstein/Ts. (DE)**
Erfinder : **Kussmaul, Ulrich, Dr.**
**Tannenweg 39a**
**D-6367 Karben 1 (DE)**

(74) Vertreter : **Urbach, Hans-Georg, Dr. et al**
**Hanauer Landstrasse 526**
**D-6000 Frankfurt am Main 61 (DE)**

**EP 0 141 053 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von 2-Chlorbenzoxazolen der Formel I

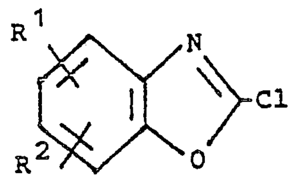

(I)

worin die Symbole $R^1$ und $R^2$ unabhängig voneinander Wasserstoff oder Halogen bedeuten, bei dem Benzoxazolinone der Formel

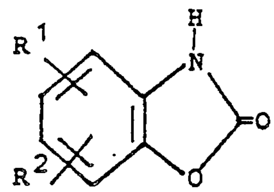

(II)

mit Phosphorpentachlorid umgesetzt werden.

Die für $R^1$ und $R^2$ gegebenenfalls stehenden Halogenatome können unabhängig voneinander vorzugsweise Fluor, Chlor oder Bromatome, insbesondere Chloratome, sein. Steht nur $R^1$ für Halogen, $R^2$ aber für Wasserstoff, so kann das Halogenatom die Positionen 4, 5, 6 oder 7 des Benzoxazols einnehmen ; sind $R^1$ und $R^2$ beide Halogenatome, so können diese in 4,5-, 4,6-, 4,7-, 5,6-, 5,7- oder 6,7-Stellung stehen. Bei Einfachsubstitution des Benzo-Kerns des Benzoxazols sind die Positionen 5 und insbesondere 6, bei Zweifachsubstitution die Stellung 5,7 besonders bevorzugt.

2-Chlorbenzoxazole der Formel I sind wertvolle Zwischenprodukte für die Synthese von Pflanzenschutzmitteln.

Die präparative Herstellung der 2-Chlorbenzoxazole der Formel I erfolgte bisher ausschließlich aus den entsprechenden 2-Mercapto-Benzoxazolen durch Austausch der Mercaptogruppe gegen Chlor unter Einsatz von Chlorierungsmitteln. In Heterocyclic Compounds, Vol. 5, R.C. Elderfield, Verlag John Wiley (New York) and Chapman and Hall (London), S. 447, wird die Möglichkeit der Herstellung von 2-Chlorbenzoxazol ausgehend von Benzoxazolon und Phosphorpentachlorid ausgeschlossen, da sich Kernchlorierungen nicht vermeiden lassen. So wird nach den Angaben in J. pr. Chemie 42, 454 (1890) 2-Chlorbenzoxazol durch Erhitzen der entsprechenden 2-Merkaptoverbindung mit Phosphorpentachlorid, das nach Möglichkeit im Unterschuß eingesetzt werden soll, ohne Lösungs- oder Verdünnungsmittel erhalten. Bei diesem Verfahren tritt gegen Ende der Reaktion Verkohlung ein ; die Ausbeuten sind sehr niedrig und es werden verschiedene z. T. schlecht abtrennbare Nebenprodukte erhalten. Andere Chlorierungsmittel, die für den Austausch der 2-Mercaptogruppe im 2-Mercaptobenzoxazol gegen Chlor verwendet werden können, sind z. B. Dischwefel-dichlorid (Boll. Sci. Fac. Chim. Ind. Bologna 23 (2-3) 89-98 (1965)), Phosgen (DE-PS 11 64 413) oder auch elementares Chlor (Am. Chemical Journal 21, 123 (1899)). Die genannten bekannten Verfahren haben gemeinsam den Nachteil, daß als Ausgangsmaterial die entsprechenden 2-Merkaptobenzoxazole verwendet werden. Bei deren Herstellung aus den entsprechenden 2-Aminophenolen sind wegen des erforderlichen Einsatzes bzw. wegen der partiellen Abspaltung von Schwefelkohlenstoff aufwendige Sicherheitsbestimmungen zu beachten, so daß der Ersatz des 2-Merkaptobenzoxazols durch billigere Ausgangsprodukte überaus wünschenswert ist. Ein weiterer Nachteil der genannten bekannten Verfahren ist der Zwangsanfall von z. T. nicht einfach zu entsorgenden und teils schwer abzutrennenden Nebenprodukten.

In neuerer Zeit ist auch bereits ein Verfahren beschrieben worden (vgl. Zeitschrift für Chemie 5 (1965), Seite 178) zur Herstellung von 2-Chlorbenzoxazol aus Benzoxazolinon. Nach den Angaben dieser Veröffentlichung soll der Austausch der Carbonylfunktion gegen Chlor durch Umsetzung des Ausgangsmaterials mit einem speziellen Chlorierungsreaganz, dem Brenzkatechylphosphortrichlorid erfolgen. Wegen gleichzeitig ablaufender Nebenreaktionen ist jedoch die Ausbeute dieses Verfahrens sehr unbefriedigend ; sie liegt nach den Angaben der Druckschrift bei nur 42 % d. Th.

Entsprechend schwierig gestaltet sich die Isolierung des gewünschten 2-Chlorbenzoxazols.

Es wurde nun gefunden, daß man überraschenderweise 2-Chlorbenzoxazole der Formel I in einfacher Weise und in guter Ausbeute durch Umsetzung von Benzoxazolinonen der Formel II mit Phosphorpentachlorid erhalten kann, wenn man darauf achtet, daß in der Reaktionsmischung dem Benzoxazolinon der Formel II stets ein gewisser Überschuß von Phosphorpentachlorid gegenübersteht.

Aus technischen Gründen ist es zur Herbeiführung eines homogenen Reaktionsgemisches und für den glatten Ablauf der Reaktion vorteilhaft, in einem Lösungs- oder Verdünnungsmittel zu arbeiten. Zweckmäßigerweise wird die Reaktion bei Temperaturen zwischen 120 und 200 °C, vorzugsweise 140 und 180 °C, ausgeführt. Als Lösungsmittel eignen sich inerte, aromatische Kohlenwasserstoffe, wie z. B. Benzol, Toluol, Xylol, technische Xylolgemische, vorzugsweise halogenierte aromatische Kohlenwasserstoffe, wie z. B. Monochlorbenzol, o-Dichlorbenzol, Trichlorbenzol, Chlortoluol aber auch nicht zuletzt Phosphoroxychlorid.

Werden Lösungsmittel eingesetzt, deren Siedetemperatur unterhalb der gewünschten Reaktionstemperatur liegt, so ist das Verfahren in an sich bekannter Weise im geschlossenen Gefäß unter Druck auszuführen.

Das wesentliche Kriterium des erfindungsgemäßen Verfahrens ist die Reaktion der Benzoxazolinone mit im Überschuß vorliegendem Phosphorpentachlorid. Dies kann man erreichen, indem man das Benzoxazolinon mit einem größeren molaren Überschuß von beispielsweise 2 bis 10 mol Phosphorpentachlorid pro mol Benzoxazolinon gemeinsam auf die Reaktionstemperatur bringt.. Vorteilhafter ist es, Phosphorpentachlorid in einem Lösungs- oder Verdünnungsmittel vorzulegen und bei der gewünschten Reaktionstemperatur das Benzoxazolinon als Feststoff, Suspension oder Lösung allmählich zuzudosieren.

Als besonders vorteilhaft hat sich bei dem erfindungsgemäßen Verfahren der Einsatz von 2 bis 10 mol, insbesondere 3 bis 5 mol, Phosphorpentachlorid pro mol umzusetzendem Benzoxazolinon der Formel II erwiesen.

Eine besonders bevorzugte Ausgestaltungsform des erfindungsgemäßen Verfahrens besteht demnach darin, daß pro mol umzusetzendem Benzoxazolinon 2 bis 10 mol, vorzugsweise 3 bis 5 mol Phosphorpentachlorid in einem inerten Lösungsmittel, vorzugsweise einem höher siedenden, halogenierten aromatischen Lösungsmittel, wie z. B. o-Dichlorbenzol auf eine Reaktiontemperatur zwischen 120 und 200 °C, vorzugsweise 140 bis 180 °C, aufgeheizt werden und portionsweise oder vorzugsweise kontinuierlich das Benzoxazolinon eingetragen wird.

Die Reaktion ist in der Regel nach kurzer Zeit beendet. Bei der Durchführung des Verfahrens nach der oben beschriebenen besonders bevorzugten Ausführungsform kann das Eintragen des Benzoxazolinons so schnell durchgeführt werden, wie es die eintretende Chlorwasserstoffentwicklung gestattet. Nach beendetem Eintragen des Benzoxazolinons ist ein Nacherhitzen von ca. 3 bis 15 Minuten zur Vervollständigung der Reaktion ausreichend.

Die Aufarbeitung des Reaktionsgemisches nach der Reaktion erfolgt in an sich bekannter Weise, vorteilhaft durch fraktionierte Destillation. Bei Einsatz eines größeren Phosphorpentachloridüberschusses ist es jedoch zweckmäßig, vor der Destillation den Reaktionsansatz kurz auf Temperaturen zwischen 0 und 30 °C abzukühlen, da bei geeigneter Wahl des Lösungsmittels, zum Beispiel bei Benutzung von o-Dichlorbenzol, hierbei der größte Teil des Phosphorpentachloridüberschusses aus dem Ansatz auskristallisiert und in besonders einfacher Weise durch Absaugen isoliert und wiederverwendet werden kann.

Nach dem erfindungsgemäßen Verfahren lassen sich aus technisch relativ leicht zugänglichen Ausgangsmaterialien in guten Ausbeuten und sehr hoher Reinheit 2-Chlorbenzoxazole der Formel I herstellen. Die Isolierung der Endprodukte erfolgt durch die technisch einfach durchzuführende fraktionierte Destillation, vorzugsweise unter Vakuum. Überschüssiges Chlorierungsmittel und eingesetztes Lösungsmittel lassen sich auf diese Weise leicht regenerieren. Das Verfahren arbeitet somit insgesamt ganz erheblich ökonomischer als bisher bekannte Verfahren zur Herstellung von 2-Chlorbenzoxazolen.

Besonders wertvoll ist das erfindungsgemäße Verfahren zur Herstellung der als Zwischenprodukte für die Herstellung von Pflanzenschutzmitteln erforderlichen 2-Chlorbenzoxazole der Formel I, in denen $R^1$ Halogen, insbesondere Chlor, bedeutet und $R^2$ für Wasserstoff steht. Dabei steht das für $R^1$ stehende Halogen, insbesondere das Chlor, vorzugsweise in 6-Stellung.

Die als Ausgangsprodukte für das erfindungsgemäße Verfahren benötigten Benzoxazolinone der Formel II sind aus den entsprechend substituierten o-Aminophenolen durch Umsetzung mit Kohlensäure-Derivaten in bekannter Weise leicht zugänglich und werden zum Teil großtechnich hergestellt.

Das als Ausgangsprodukt für die Herstellung von 2,6-Dichlorbenzoxazol erforderliche 6-Chlorbenzoxazolin-2-on braucht bei der Durchführung des erfindungsgemäßen Verfahrens nicht in isolierter Form eingesetzt zu werden, sondern kann in Form eines bei der Kernchlorierung von Benzoxazolin-2-on in einem organischen Lösungsmittel anfallenden rohen Reaktionsgemisches eingesetzt werden. Dieses Reaktionsgemisch liegt in der Regel in Form einer Suspension vor.

Die Kernchlorierung von Benzoxazolin-2-on, d. h. seine Überführung in 6-Chlorbenzoxazolin-2-on, wird mit einem geeigneten Chlorierungsmittel, normalerweise elementares Chlor oder Sulfurylchlorid, in einem inerten organischen Lösungsmittel bei Temperaturen von 0 bis 150 °C, vorzugsweise 20 bis 120 °C, durchgeführt. Als geeignete inerte organische Lösungsmittel kommen vor allem die bereits genannten halogenierten aromatischen Kohlenwasserstoffe, vorzugsweise o-Dichlorbenzol, in Betracht. Bei der Kernchlorierung von Benzoxazolin-2-on zu 6-Chlorbenzoxazolin-2-on wird das Kernchlorierungsmittel in 1,0- bis 1,3-molarer, vorzugsweise 1,0- bis 1,1-molarer Menge, eingesetzt.

Das bei der Kernchlorierung von Benzoxazolin-2-on anfallende Reaktionsgemisch braucht für die anschließende Umsetzung mit überschüssigem Phosphorpentachlorid nicht aufgearbeitet oder gereinigt

zu werden, sondern kann direkt nach dem erfindungsgemäßen Verfahren mit überschüssigen Phosphorpentachlorid umgesetzt werden. Dies kann z. B. dadurch geschehen, daß man das Phosphorpentachlorid dem Kernchlorierungsgemisch zugibt oder vorteilhaft dadurch, daß überschüssiges Phosphorpentachlorid in einem inerten organischen Lösungsmittel vorgelegt und bei der gewünschten Reaktionstemperatur das Kernchlorierungsgemisch allmählich zudosiert wird.

Das für die Kernchlorierung benötigte Benzoxazolin-2-on braucht ebenfalls nicht als Reinsubstanz eingesetzt zu werden, sondern kann für die Kernchlorierung in Form einer rohen Cyclisierungslösung eingesetzt werden, die man bei der Cyclisierung von 2-Aminophenol mit einem Kohlensäurederivat in einem halogenierten aromatischen Kohlenwasserstoff, vorzugsweise o-Dichlorbenzol, erhält.

Geeignete Kohlensäurederivate für diese Cyclisierung sind z. B. Carbamidsäurechlorid, Urethane der Formel $O{=}C(NH_2)OR$, Chlorameisensäureester der Formel $O{=}C(Cl)OR$, und Kohlensäureester der Formeln $O{=}C(OR)_2$ oder $O{=}C(OR)OR'$, wobei R bzw. R' in den vorgenannten Formeln insbesondere Alkyl, vorzugsweise Alkyl mit 1 bis 4 C-Atomen, bedeutet. Besonders geeignete Kohlensäurederivate für die Cyclisierung sind jedoch Phosgen und Harnstoff.

Diese Cyclisierung von 2-Aminophenol mit Kohlensäurederivaten, wie z. B. Phosgen oder Harnstoff, zu Benzoxazolin-2-on, wird unter Schutzgasatmosphäre bei Temperaturen oberhalb 100 °C, z. B. von 100 bis 190 °C, durchgeführt, wobei pro mol 2-Aminophenol 1 bis 1,3 mol des Kohlensäurederivates eingesetzt werden. Gegebenenfalls kann es bei der Verwendung von Harnstoff als Cyclisierungsmittel zweckmäßig sein, vor der folgenden Kernchlorierung einen Teil des Lösungsmittels aus der Cyclisierungslösung abzudestillieren, um das als Nebenprodukt entstehende Ammoniak vollständig aus dem Reaktionsgemisch zu entfernen.

Eine besonders bevorzugte Ausgestaltungsform des erfindungsgemäßen Verfahrens zur Herstellung von 2,6-Dichlorbenzoxazol besteht demnach darin, daß Benzoxazolin-2-on, gegebenenfalls in Form eines rohen Cyclisierungsgemisches, in einem halogenierten aromatischen Kohlenwasserstoff, vorzugsweise in o-Dichlorbenzol, mit 1,0- bis 1,3-molaren, vorzugsweise 1,0- bis 1,1-molaren Mengen eines Kernchlorierungsmittels umgesetzt und die erhaltene Suspension anschließend zu einer auf 120 bis 200 °C, vorzugsweise 140 bis 180 °C aufgeheizten Lösung der 2- bis 10fach, vorzugsweise 3- bis 5fach molaren Menge Phosphorpentachlorid in demselben Lösungsmittel portionsweise oder vorzugsweise kontinuierlich zudosiert wird. Die Zudosierung wird auch in diesem Fall so schnell durchgeführt, daß die Reaktionstemperatur nicht unterschritten wird und der entstehende Chlorwasserstoff abgeführt werden kann. Nach beendeter Zudosierung ist auch in diesem Fall ein Nacherhitzen von ca. 3 bis 15 Minuten zur Vervollständigung der Reaktion ausreichend.

Auch bei der Herstellung von 2,6-Dichlorbenzoxazol, ausgehend von Benzoxazolin-2-on nach dem beschriebenen zweistufigen Verfahren, fallen als Nebenprodukte nur die leicht zu absorbierenden gasförmigen Verbindungen Chlorwasserstoff und (nur bei Verwendung von Sulfurylchlorid als Kernchlorierungsmittel) Schwefeldioxid sowie das bei der Destillation einfach abzutrennende Phosphoroxychlorid an. Auch das zweistufige Verfahren arbeitet somit insgesamt ganz erheblich ökonomischer und ökologisch unbedenklicher als bisher bekannte Verfahren zur Herstellung von 2,6-Dichlorbenzoxazol.

Bei dem zweistufigen Verfahren zur Herstellung von 2,6-Dichlorbenzoxazol ist die hohe Produktreinheit und die hohe Ausbeute im Vergleich zu der bei der Umsetzung eines reinen isolierten 6-Chlorbenzoxazolin-2-ons mit Phosphorpentachlorid erhaltenen Ausbeute besonders überraschend. In der Literatur werden für die Herstellung von 6-Chlorbenzoxazolin-2-on durch Kernchlorierung von Benzoxazolin-2-on bei Verwendung von Chlor Ausbeuten um 90 % und bei der Verwendung von Sulfurylchlorid eine Ausbeute von 81 % angegeben. Demzufolge sollte man bei der weiteren Umsetzung eines rohen Chlorierungsgemisches, verglichen mit der Umsetzung der isolierten Reinsubstanz, eine entsprechend verminderte Ausbeute und eine durch isomere Nebenprodukte verminderte Produktreinheit beobachten. Es war nicht vorherzusehen, daß man nach dem erfindungsgemäßen Verfahren bei Verwendung von 6-Chlorbenzoxazolin-2-on in Form eines rohen Chlorierungsgemisches 2,6-Dichlorbenzoxazol in fast gleich hoher Reinheit (Gehalt an 2,6-Dichlorbenzoxazol über 99 %, gaschromatographisch bestimmt) und mit einer Minderausbeute von nur 2 % gegenüber dem Verfahren erhält, das von reinem 6-Chlorbenzoxazolin-2-on ausgeht. Setzt man bei der Herstellung von 2,6-Dichlorbenzoxazol anstelle von Benzoxazolin-2-on ein rohes Cyclisierungsgemisch in einem inerten organischen Lösungsmittel von 2-Aminophenol und einem Kohlensäurederivat ein, dann wird die Ausbeute an 2,6-Dichlorbenzoxazol etwas verringert, seine Reinheit liegt aber immer noch über 95 %.

Die nachfolgenden Ausführungsbeispiele veranschaulichen die Durchführung des erfindungsgemäßen Verfahrens : (Die Abkürzung GC bedeutet Gaschromatogramm).

## Beispiel 1

1 l o-Dichlorbenzol und 625 g (3 mol) Phosphorpentachlorid werden auf 160 °C erhitzt, dann werden im Laufe von 60 Minuten 169,5 g (1 mol) 6-Chlorbenzoxazolin-2-on in kleinen Portionen fest eingetragen, wobei nach jeder Portion kurzzeitig starke Chlorwasserstoffentwicklung auftritt. Es wird weitere 15 Minuten bei 150-160 °C nachgerührt, dann auf 0 °C abgekühlt, wobei der größte Teil des $PCl_5$-Überschusses auskristallisiert. Man saugt ab, wäscht den Niederschlag von Phosphorpentachlorid mit etwas kaltem o-Dichlorbenzol und fraktioniert das Filtrat unter vermindertem Druck. Zuerst geht

Phosphoroxychlorid über, dann o-Dichlorbenzol zusammen mit dem restlichen Überschuß an Phosphorpentachlorid und zuletzt, bei etwa 110 °C und 17,3 mbar, 2,6-Dichlorbenzoxazol. Man erhält ca. 136 g 2,6-Dichlorbenzoxazol vom Schmelzpunkt 48-49 °C. Reinheit nach GC 99,7 %. Ausbeute : 72 % der Theorie.

Das wiedergewonnene Phosphorpentachlorid und o-Dichlorbenzol kann beim nächsten Ansatz wieder eingesetzt werden.

Beispiel 2

Verfährt man wie im Beispiel 1, setzt aber statt 625 g (3 mol) 1 040 g (5 mol) Phosphorpentachlorid ein, so erhält man 145 g 2,6-Dichlorbenzoxazol entsprechend einer Ausbeute von 77 % d. Th.

Beispiel 3

Verfährt man wie im Beispiel 1, setzt aber wiedergewonnenes, ca. 90 %iges o-dichlorbenzolfeuchtes Phosphorpentachlorid und wiedergewonnenes o-Dichlorbenzol ein, so erhält man 2,6-Dichlorbenzoxazol in gleicher Ausbeute und Reinheit wie im Beispiel 1.

Beispiel 4

Verfährt man wie im Beispiel 1, legt jedoch nur 500 ml o-Dichlorbenzol vor und dosiert das 6-Chlorbenzoxazolin-2-on als Suspension in 500 ml o-Dichlorbenzol zu, so erhält man 2,6-Dichlorbenzoxazol in gleicher Ausbeute und Reinheit wie im Beispiel 1.

Beispiel 5

Verfährt man wie im Beispiel 1, setzt jedoch anstelle von 6-Chlorbenzoxazolin-2-on die gleiche Menge 5-Chlorbenzoxazolin-2-on ein, so erhält man ca. 130 g entsprechend 69 % d. Th. an 2,5-Dichlorbenzoxazol, Fp. 46-48 °C.

Beispiel 6

Analog Beispiel 1 werden anstelle von 6-Chlorbenzoxazolin-2-on 204 g 5,7-Dichlorbenzoxazolin-2-on umgesetzt. Aus dem nach dem Abdestillieren des o-Dichlorbenzols verbleibenden Rückstand wird durch Umkristallisieren aus Toluol oder Ligroin das 2,5,7-Trichlorbenzoxazol vom Schmelzpunkt 140-142 °C in einer Ausbeute von 68 % d. Th. gewonnen.

Beispiel 7

500 ml o-Dichlorbenzol und 625 g (3 mol) Phosphorpentachlorid werden auf 150 °C erhitzt, dann werden im Laufe von 30 Minuten bei einer Innentemperatur von 140-150 °C 151 g (1 mol) Benzoxazolin-2-on, suspendiert in 500 ml o-Dichlorbenzol, zudosiert. Man rührt 5 Minuten bei 140 °C nach und kühlt dann rasch auf 5 °C ab, wobei der größte Teil des Phosphorpentachloridüberschusses auskristallisiert. Man saugt ab, wäscht den Niederschlag von Phosphorpentachlorid mit etwas kaltem o-Dichlorbenzol und fraktioniert das Filtrat unter vermindertem Druck. Zuerst geht Phosphoroxychlorid über, dann o-Dichlorbenzol zusammen mit dem restlichen Überschuß an Phosphorpentachlorid und zuletzt ca. 115 g (75 % d. Th.) 2-Chlorbenzoxazol vom Kp 104-107 °C bei 30,6 mbar.

Beispiel 8

In 600 ml o-Dichlorbenzol werden bei Raumtemperatur 135 g (1 mol) trockenes Benzoxazolin-2-on suspendiert. Im Verlaufe von 1 h werden 142 g (1,05 mol) Sulfurylchlorid zugetropft, wobei die Innentemperatur auf ca. 40 °C ansteigt. Es wird weitere 10 h bei 40 °C gerührt, dann im Verlaufe von 3 h auf 90 °C erhitzt und 1 h nachgerührt. Man erhält das Rohgemisch 1.

1 040 g (5 mol) Phosphorpentachlorid werden mit 400 ml o-Dichlorbenzol auf 150 bis 160 °C erhitzt. Dazu wird im Verlauf von 2 h das Rohgemisch 1 unter Heizen so zudosiert, daß die Innentemperatur 150 °C nicht unterschreitet. Nach Beendigung der Zugabe wird 10 min nachgerührt, dann auf 10 °c abgekühlt, das ausgefallene Phosphorpentachlorid abgesaugt und mit etwas kaltem o-Dichlorbenzol nachgewaschen. Bei der Fraktionierung des Filtrats unter vermindertem Druck über eine 15-cm-Kolonne mit Raschig-Ringen geht zuerst Phosphoroxychlorid über, dann o-Dichlorbenzol zusammen mit dem restlichen Überschuß Phosphorpentachlorid und zuletzt, bei etwa 110 °C bei 17,3 mbar 2,6-Dichlorbenzoxazol. Die Ausbeute beträgt 141 g entsprechend 75 % der Theorie, bezogen auf eingesetztes Benzoxazolin-2-on. Reinheit des Produkts nach GC 99,3 %. Gehalt an 2-Chlorbenzoxazol 0,3 %, an 2,5-Dichlorbenzoxazol 0,1 %.

Beispiel 9

400 ml o-Dichlorbenzol und 1 040 g (5 mol) Phosphorpentachlorid werden auf 150 bis 160 °C erhitzt, dann werden im Laufe von 2 h 169,5 g (1 mol) 6-Chlorbenzoxazolin-2-on, suspendiert in 600 ml o-Dichlorbenzol, unter Heizen so zudosiert, daß die Innentemperatur 150 °C nicht unterschreitet. Nach Beendigung der Zugabe wird 10 min nachgerührt. Die Aufarbeitung analog Beispiel 8 ergibt eine Ausbeute von 145 g 2,6-Dichlorbenzoxazol entsprechend 77 % der Theorie. Reinheit nach GC 99,7 %.

Beispiel 10

Verfährt man wie in Beispiel 8, setzt aber in der zweiten Stufe die entsprechenden Mengen wiedergewonnenes Phosphorpentachlorid und o-Dichlorbenzol ein, so erhält man 2,6-Dichlorbenzoxazol in gleicher Ausbeute und Reinheit.

Beispiel 11

In 600 ml o-Dichlorbenzol werden 135 g (1 mol) trockenes Benzoxazolin-2-on suspendiert. Bei einer Temperatur von 100 °C werden im Verlauf von 2 h 75 g (1,05 mol) Chlor eingeleitet. Der entweichende Chlorwasserstoff wird in Wasser absorbiert.

Das Rohgemisch wird analog Beispiel 8 mit Phosphorpentachlorid umgesetzt und aufgearbeitet. Man erhält 138 g (73 % der Theorie) 2,6-Dichlorbenzoxazol in vergleichbarer Reinheit.

Beispiel 12

In 350 ml o-Dichlorbenzol werden 109 g (1 mol) 2-Aminophenol und 63 g (1,05 mol) Harnstoff suspendiert und unter Stickstoff 3 h auf 150 bis 160 °C erhitzt, wobei Ammoniak entweicht. Durch leichte Druckerniedrigung werden ca. 100 ml des Lösungsmittels abdestilliert und anschließend 300 ml frisches o-Dichlorbenzol zugesetzt. Bei 110 °C Innentemperatur werden dann innerhalb von 1 bis 2 h 78 g (1,1 mol) Chlor eingeleitet, wobei Chlorwasserstoff entweicht. Das Rohgemisch wird analog Beispiel 8 mit Phosphorpentachlorid umgesetzt und aufgearbeitet. Man erhält 115 g (61 % der Theorie) 2,6-Dichlorbenzoxazol. Reinheit nach GC 96,8 %.

Beispiel 13

In 350 ml o-Dichlorbenzol werden 109 g (1 mol) 2-Aminophenol suspendiert und unter Stickstoff auf 120 °C erhitzt. Bei dieser Temperatur werden in ca. 2 h 119 g (1,2 mol) Phosgen eingeleitet. Der Ansatz wird auf 40 °C abgekühlt. Dann werden im Laufe von einer halben Stunde 162 g (1,2 mol) Sulfurylchlorid zugetropft, 10 h bei 40 °C, dann 1 h bei 90 °C nachgerührt. Das Rohgemisch wird analog Beispiel 8 mit Phosphorpentachlorid umgesetzt und aufgearbeitet. Man erhält 92 g (49 % der Theorie) 2,6-Dichlorbenzoxazol. Reinheit nach GC 95,9 %.

**Patentansprüche**

1. Verfahren zur Herstellung von 2-Chlorbenzoxazolen der Formel I

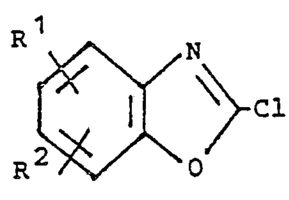

(I)

worin die Symbole $R^1$ und $R^2$ unabhängig voneinander Wasserstoff oder Halogen bedeuten, dadurch gekennzeichnet, daß Benzoxazolinone der Formel II

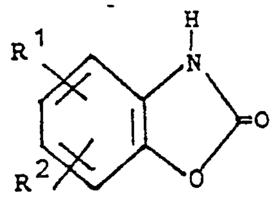

(II)

mit im Überschuß vorliegendem Phosphorpentachlorid umgesetzt werden.

2. Verfahren nach Anspruch 1, dadurch gekennzeichnet, daß pro mol des Benzoxazolinons II 2 bis 10 mol Phosphorpentachlorid eingesetzt werden.

3. Verfahren nach den Ansprüchen 1 und/oder 2, dadurch gekennzeichnet, daß das Phosphorpentachlorid vorgelegt und das Benzoxazolinon anschließend nach und nach zugesetzt wird.

4. Verfahren nach einem oder mehreren der Ansprüche 1 bis 3, dadurch gekennzeichnet, daß die Umsetzung in einem inerten Lösungsmittel durchgeführt wird.

5. Verfahren nach einem oder mehreren der Ansprüche 1 bis 4, dadurch gekennzeichnet, daß die Reaktion bei Temperaturen von 120 bis 200 °C durchgeführt wird.

6. Verfahren nach einem oder mehreren der Ansprüche 1 bis 5, dadurch gekennzeichnet, daß ein Benzoxazolinon der Formel II eingesetzt wird, in dem $R^1$ Halogen, $R^2$ Wasserstoff bedeutet.

7. Verfahren nach einem oder mehreren der Ansprüche 1 bis 6, dadurch gekennzeichnet, daß ein Benzoxazolinon der Formel II eingesetzt wird, in dem $R^1$ Chlor, $R^2$ Wasserstoff bedeutet.

8. Verfahren nach einem oder mehreren der Ansprüche 1 bis 7, dadurch gekennzeichnet, daß bei der Herstellung von 2,6-Dichlorbenzoxazol als Ausgangsprodukt ein Reaktionsgemisch eingesetzt wird, das durch Kernchlorierung von Benzoxazolin-2-on in einem inerten organischen Lösungsmittel erhalten worden ist.

9. Verfahren nach einem oder mehreren der Ansprüche 1 bis 8, dadurch gekennzeichnet, daß bei der Herstellung von 2,6-Dichlorbenzoxazol als Ausgangsprodukt ein Reaktionsgemisch eingesetzt wird, das in einem inerten organischen Lösungsmittel durch Cyclisierung von 2-Aminophenol mit einem Kohlensäurederivat und anschließende Kernchlorierung erhalten worden ist.

10. Verfahren nach einem oder mehreren der Ansprüche 1 bis 9, dadurch gekennzeichnet, daß die Umsetzung, sowie gegebenenfalls die vorausgehende Kernchlorierung und gegebenenfalls auch die vorausgehende Cyclisierung, in o-Dichlorbenzol als Lösungsmittel durchgeführt werden.

**Claims**

1. Process for the preparation of 2-chlorobenzoxazoles of the formula I

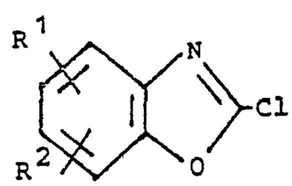

(I)

in which the symbols $R^1$ and $R^2$, independently of one another, denote hydrogen or halogen, characterised in that benzoxazolinones of the formula II

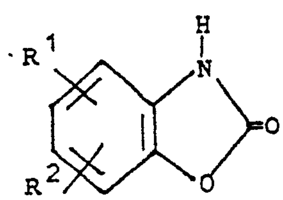

(II)

are reacted with phosphorus pentachloride which is present in excess.

2. Process according to Claim 1, characterised in that 2 to 10 moles of phosphorus pentachloride are used per mole of the benzoxazolinone II.

3. Process according to Claims 1 and/or 2, characterised in that the phosphorus pentachloride is intitially introduced, and the benzoxazolinone is then added portionwise.

4. Process according to one or several of Claims 1 to 3, characterised in that the reaction is carried out in an inert solvent.

5. Process according to one or several of Claims 1 to 4, characterised in that the reaction is carried out at temperatures from 120 to 200 °C.

6. Process according to one or several of Claims 1 to 5, characterised in that a benzoxazolinone of the formula II in which $R^1$ denotes halogen and $R^2$ denotes hydrogen is used.

7. Process according to one or several of Claims 1 to 6, characterised in that a benzoxazolinone of the formula II in which $R^1$ denotes chlorine and $R^2$ denotes hydrogen is used.

8. Process according to one or several of Claims 1 to 7, characterised in that in the preparation of 2,6-dichlorobenzoxazole the starting product used is a reaction mixture which has been obtained by ring chlorination of benzoxazolin-2-one in an inert organic solvent.

9. Process according to one or several of claims 1 to 8, characterised in that in the preparation of 2,6-dichlorobenzoxazole the starting product used is a reaction mixture which has been obtained in an inert organic solvent by cyclization of 2-aminophenol with a carbonic acid derivative and subsequent ring chlorination.

10. Process according to one or several of claims 1 to 9, characterised in that the reaction and, if applicable, the preceding ring chlorination and, if applicable, also the preceding cyclization, are carried out in o-dichlorobenzene as solvent.

**Revendications**

1. Procédé pour préparer des chloro-2 benzoxazoles répondant à la formule 1 :

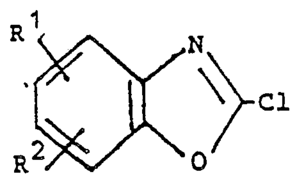

(I)

dans laquelle $R^1$ et $R^2$ représentent chacun, indépendamment l'un de l'autre, l'hydrogène ou un halogène, procédé caractérisé en ce qu'on fait réagir des benzoxazolinones de formule II :

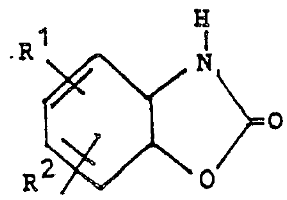

(II)

avec du pentachlorure de phosphore mis en jeu en excès.

2. Procédé selon la revendication 1 caractérisé en ce qu'on met en jeu, par mole de la benzoxazolinone (II), de 2 à 10 mol de pentachlorure de phosphore.

3. Procédé selon l'une des revendications 1 et 2, caractérisé en ce que le pentachlorure de phosphore est mis en place dès le départ et la benzoxazolinone est ensuite ajoutée peu à peu.

4. Procédé selon l'une quelconque des revendications 1 à 3, caractérisé en ce que la réaction est effectuée dans un solvant inerte.

5. Procédé selon l'une quelconque des revendications 1 à 4, caractérisé en ce que la réaction est effectuée à des températures de 120 à 200 °C.

6. Procédé selon l'une quelconque des revendications 1 à 5, caractérisé en ce qu'on utilise une benzoxazolinone de formule II dans laquelle $R^1$ représente un halogène et $R^2$ l'hydrogène.

7. Procédé selon l'une quelconque des revendications 1 à 6, caractérisé en ce qu'on utilise une benzoxazolinone de formule II dans laquelle $R^1$ représente le chlore et $R^2$ l'hydrogène.

8. Procédé selon l'une quelconque des revendications 1 à 7, caractérisé en ce que, dans la préparation du dichloro-2,6 benzoxazole, on utilise comme produit de départ un mélange réactionnel que l'on a obtenu en chlorant sur le noyau la benzoxazolinone-2 dans un solvant organique inerte.

9. Procédé selon l'une quelconque des revendications 1 à 8, caractérisé en ce que, dans la préparation du dichloro-2,6 benzoxazole, on utilise comme produit de départ un mélange réactionnel que l'on a obtenu, dans un solvant organique inerte, par cyclisation de l'amino-2 phénol avec un dérivé de l'acide carbonique, puis chloration sur le noyau.

10. Procédé selon l'une quelconque des revendications 1 à 9, caractérisé en ce qu'on effectue la réaction, et éventuellement aussi la chloration préalable sur le noyau et, éventuellement, la cyclisation préalable, dans un solvant qui est l'o-dichlorobenzène.